# EUROPEAN PATENT APPLICATION

(11) **EP 2 351 560 A1**
(43) Date of publication of application: **03.08.2011**
(21) Application number: 10179079.8
(22) Date of filing: 02.01.2006
(51) Int. Cl.: A61K 31/137, A61K 31/661, A61K 31/381, A61K 31/397, A61P 31/14

(54) **Treatment Of HCV infections with FTY720**

(30) Priority: 04.01.2005 GB 0500020
(62) Divisional of application: 06700482.0
(71) Applicant: Novartis AG, 4056 Basel (CH); Novartis Pharma GmbH, 1230 Wien (AT)
(72) Inventor: Brinkmann, Volker, 79102, Freiburg (DE); Feutren, Gilles, 68100, Mulhouse (FR)
(74) Representative: Rouquayrol, Céline Hélène

(57) **Abstract**

S1P receptor agonists are useful for the treatment of hepatitis C or chronic hepatitis C (HCV).

## Description

The present invention relates to the use of an S1P receptor modulator or agonist in hepatitis C or chronic hepatitis C.

S1 P receptor modulators or agonists are typically sphingosine analogues, such as 2-substituted 2-amino- propane-1,3-diol or 2-amino-propanol derivatives, e. g. a compound comprising a group of formula X 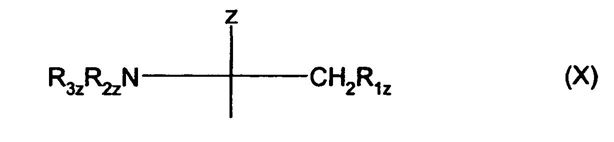 wherein Z is H, C₁₋₆alkyl, C₂₋₆alkenyl, C₂₋₆alkynyl, phenyl, phenyl substituted by OH, C₁₋₆alkyl substituted by 1 to 3 substituents selected from the group consisting of halogen, C₃₋₈cycloalkyl, phenyl and phenyl substituted by OH, or CH₂-R_{4z}, wherein R_{4z} is OH, acyloxy or a residue of formula (a) 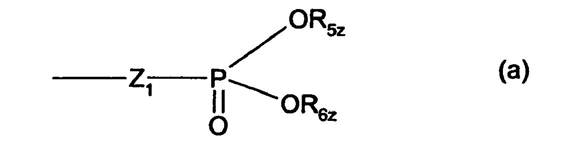 wherein Z₁ is a direct bond or O preferably O;
each of R_{5z} and R_{6z}, independently, is H, or C₁₋₄alkyl optionally substituted by 1, 2 or 3 halogen atoms;
R_{1z} is OH, acyloxy or a residue of formula (a); and each of R_{2z} and R_{3z} independently, is H, C₁₋₄alkyl or acyl.

Group of formula X is a functional group attached as a terminal group to a moiety which may be hydrophilic or lipophilic and comprise one or more aliphatic, alicyclic, aromatic and/or heterocyclic residues, to the extent that the resulting molecule wherein at least one of Z and R_{1z} is or comprises a residue of formula (a), signals as an agonist at one of more sphingosine-1-phosphate receptor.

S1P receptor modulators or agonists are compounds which signal as agonists at one or more sphingosine-1 phosphate receptors, e.g. S1P1 to S1P8. Agonist binding to a S1P receptor may e.g. result in dissociation of intracellular heterotrimeric G-proteins into Gα-GTP and Gβy-GTP, and/or increased phosphorylation of the agonist-occupied receptor and activation of downstream signaling pathways/kinases.

The binding affinity of S1P receptor agonists or modulators to individual human S1P receptors may be determined in following assay:
S1P receptor agonist or modulator activities of compounds are tested on the human S1P receptors S1P₁. S1P₂, S1P₃, S1P₄ and S1P₅. Functional receptor activation is assessed by quantifying compound induced GTP [γ-³⁵S] binding to membrane protein prepared from transfected CHO or RH7777 cells stably expressing the appropriate human S1 P receptor.

The assay technology used is SPA (scintillation proximity based assay). Briefly, DMSO dissolved compounds are serially diluted and added to SPA- bead (Amersham-Pharmacia) immobilised S1P receptor expressing membrane protein (10-20µg/well) in the presence of 50 mM Hepes, 100 mM NaCl, 10 mM MgCl₂, 10 µM GDP, 0.1% fat free BSA and 0.2 nM GTP [γ-³⁵S] (1200 Ci/mmol). After incubation in 96 well microtiterplates at RT for 120 min, unbound GTP [γ-³⁵S] is separated by a centrifugation step. Luminescence of SPA beads triggered by membrane bound GTP [γ-³⁵S] is quantified with a TOPcount plate reader (Packard). EC₅₀s are calculated using standard curve fitting software. In this assay, the S1P receptor modulators or agonists preferably have a binding affinity to S1P receptor <50 nM.

Preferred S1P receptor agonists or modulators are e.g. compounds which in addition to their S1P binding properties also have accelerating lymphocyte homing properties, e.g. compounds which elicit a lymphopenia resulting from a re-distribution, preferably reversible, of lymphocytes from circulation to secondary lymphatic tissue, without evoking a generalized immunosuppression. Naive cells are sequestered; CD4 and CD8 T-cells and B-cells from the blood are stimulated to migrate into lymph nodes (LN) and Peyer's patches (PP).

The lymphocyte homing property may be measured in following Blood Lymphocyte Depletion assay:
A S1 P receptor agonist or modulator or the vehicle is administered orally by gavage to rats.
Tail blood for hematological monitoring is obtained on day -1 to give the baseline individual values, and at 2, 6, 24, 48 and 72 hours after application. In this assay, the S1 P receptor agonist or modulator depletes peripheral blood lymphocytes, e.g. by 50%, when administered at a dose of e.g. < 20 mg/kg.

Examples of appropriate S1 P receptor modulators or agonists are, for example:
- Compounds as disclosed in EP627406A1, e.g. a compound of formula I 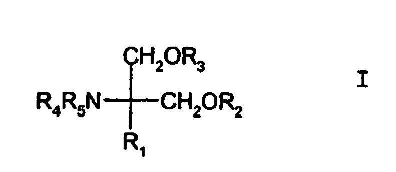 wherein R₁ is a straight- or branched (C₁₂₋₂₂)chain
- which may have in the chain a bond or a hetero atom selected from a double bond, a triple bond, O, S, NR₆, wherein R₆ is H, C₁₋₄alkyl, aryl-C₁₋₄alkyl, acyl or (C₁₋₄alkoxy)carbonyl, and carbonyl, and/or
- which may have as a substituent C₁₋₄alkoxy, C₂₋₄alkenyloxy, C₂₋₄alkynyloxy, aryLC₁₋₄alkyloxy, acyl, C₁₋₄alkylamino, C₁₋₄alkylthio, acylamino, (C₁₋₄alkoxy)carbonyl, (C₁₋₄alkoxy)-carbonylamino, acyloxy, (C₁₋₄alkyl)carbamoyl, nitro, halogen, amino, hydroxyimino, hydroxy or carboxy; or
R₁ is
- a phenylalkyl wherein alkyl is a straight- or branched (C₆₋₂₀)carbon chain; or
- a phenylalkyl wherein alkyl is a straight- or branched (C₁₋₃₀)carbon chain wherein said phenylalkyl is substituted by
- a straight- or branched (C₆₋₂₀)carbon chain optionally substituted by halogen,
- a straight- or branched (C₆₋₂₀)alkoxy chain optionally substitued by halogen,
- a straight- or branched (C₆₋₂₀)alkenyloxy,
- phenyl-C₁₋₁₄alkoxy, halophenyl-C₁₋₄alkoxy, phenyl-C₁₋₁₄alkoxy-C₁₋₁₄alkyl, phenoxy-C₁₋₄alkoxy or phenoxy-C₁₋₄alkyl,
- cycloalkylalkyl substituted by C₆₋₂₀alkyl,
- heteroarylalkyl substituted by C₆₋₂₀alkyl,
- heterocyclic C₆₋₂₀alkyl or
- heterocyclic alkyl substituted by C₂₋₂₀alkyl,
and wherein
the alkyl moiety may have
- in the carbon chain, a bond or a heteroatom selected from a double bond, a triple bond, O S, sulfinyl, sulfonyl, or NR₆, wherein R₆ is as defined above, and
- as a substituent C₁₋₄alkoxy, C₂₋₄alkenyloxy, C₂₋₄alkynyloxy, arylC₁₋₄alkyloxy, acyl, C₁₋₄alkylamino, C₁₋₄alkylthio, acylamino, (C₁₋₄alkoxy)carbonyl, (C₁₋₄alkoxy)carbonylamino, acyloxy, (C₁₋₄alkyl)carbamoyl, nitro, halogen, amino, hydroxy or carboxy, and
each of R₂, R₃, R₄ and R₅, independently, is H, C₁₋₄ alkyl or acyl
or a pharmaceutically acceptable salt or hydrate thereof;
- Compounds as disclosed in EP 1002792A1, e.g. a compound of formula II 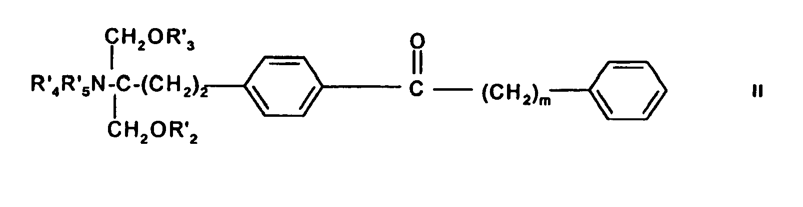
wherein m is 1 to 9 and each of R'₂, R'₃, R'₄ and R'₅, independently, is H, C₁₋₆alkyl or acyl, or a pharmaceutically acceptable salt or hydrate thereof;
- Compounds as disclosed in EP0778263 A1, e.g. a compound of formula III 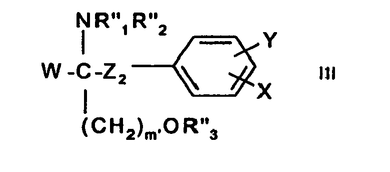
wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH;
X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆alkyl, OH, C₁₋₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₋₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
each of p and q, independently, is an integer of 1 to 20, with the proviso of 6≤+q≤23, m' is 1, 2 or 3, n is 2 or 3,
each of R"₁, R"₂, R"₃ and R"₄, independently, is H, C₁₋₄alkyl or acyl,
or a pharmaceutically acceptable salt or hydrate thereof,
- Compounds as disclosed in WO02/18395, e.g. a compound of formula IVa or IVb 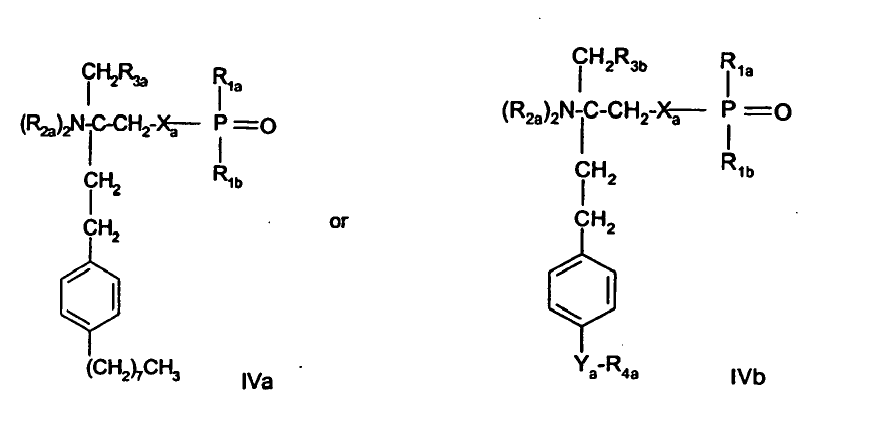
wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄₋₁₄)alkyl or (C₄₋₁₄)alkenyl;
or a pharmaceutically acceptable salt or hydrate thereof;
- Compounds as disclosed in WO 02/076995, e.g. a compound of formula V 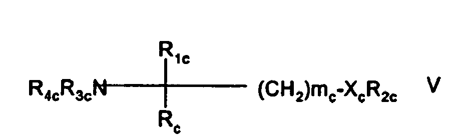
wherein
m_{c} is 1, 2 or 3;
X_{c} is O or a direct bond;
R_{1c} is H; C₁₋₆ alkyl optionally substituted by OH, acyl, halogen, C₃₋₁₀cycloalkyl, phenyl or
hydroxy-phenylene; C₂₋₆alkenyl; C₂₋₆alkynyl; or phenyl optionally substituted by OH;
R_{2c} is 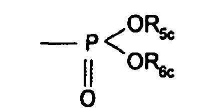 wherein R_{5c} is H or C₁₋₄alkyl optionally substituted by 1, 2 or 3 halogen atoms, and R_{6c} is H or C₁₋₄alkyl optionally substituted by halogen;
each of R_{3c} and R_{4c}, independently, is H, C₁₋₄alkyl optionally substituted by halogen, or acyl,
and
R_{c} is C₁₃₋₂₀alkyl which may optionally have in the chain an oxygen atom and which may optionally be substituted by nitro, halogen, amino, hydroxy or carboxy; or a residue of formula (a) 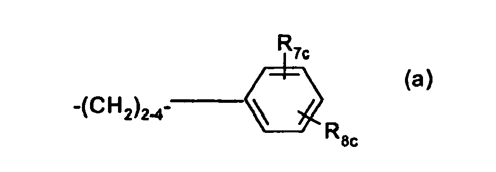 wherein R_{7c} is H, C₁₋₄alkyl or C₁₋₄alkoxy, and R_{8c} is substituted C₁₋₂₀alkanoyl, phenylC₁₋₁₄alkyl wherein the C₁₋₁₄alkyl is optionally substituted by halogen or OH, cycloalkylC₁₋₁₄alkoxy or phenylC₁₋₁₄alkoxy wherein the cycloalkyl or phenyl ring is optionally substituted by halogen, C₁₋₄alkyl and/or C₁₋₄alkoxy, phenylC₁₋₁₄alkoxy-C₁₋₁₄alkyl, phenoxyC₁₋₁₄alkoxy or phenoxyC₁₋₁₄alkyl,
R_{c} being also a residue of formula (a) wherein R_{8c} is C₁₋₁₄alkoxy when R_{1c} is C₁-4alkyl, C₂₋₆alkenyl or C₂₋₆alkynyl,
or a compound of formula VI 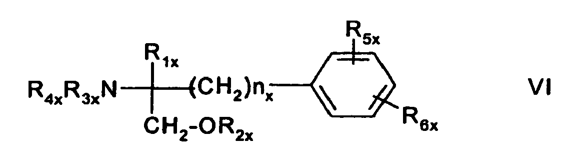 wherein
nₓ is 2, 3 or 4
R₁ₓ is H; C₁₋₆alkyl optionally substituted by OH, acyl, halogen, cycloalkyl, phenyl or hydroxy-phenylene; C₂₋₆alkenyl: C₂₋₆alkynyl; or phenyl optionally substituted by OH;
R₂ₓ is H, C₁₋₄ alkyl or acyl
each of R₃ₓ and R₄ₓ, independently is H, C₁₋₄alkyl optionally substituted by halogen or acyl, R₅ₓ is H, C₁₋₄alkyl or C₁₋₄alkoxy, and
R₆ₓ is C₁₋₂₀ alkanoyl substituted by cycloalkyl; cyloalkylC₁₋₁₄alkoxy wherein the cycloalkyl ring is optionally substituted by halogen, C₁₋₄alkyl and/or C₁₋₄alkoxy; phenylC₁₋₁₄alkoxy
wherein the phenyl ring is optionally substituted by halogen, C₁₋₄alkyl and/or C₁₋₄alkoxy, R₆ₓ being also C₄-₁₄alkoxy when R₁ₓ is C₂₋₄alkyl substituted by OH, or pentyloxy or hexyloxy
when R₁ₓ is C₁₋₄akyl, provided that R₆ₓ is other than phenyl-butylenoxy when either R₅ₓ is H or R₁ₓ is methyl, or a pharmaceutically acceptable salt or hydrate thereof;
- Compounds as disclosed in WO02/06268A1. e.g. a compound of formula VII 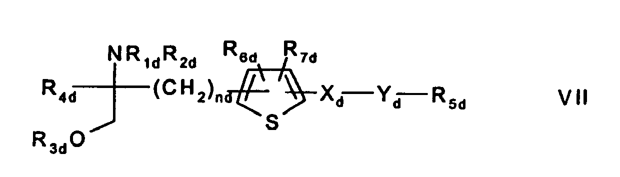
wherein each of R_{1d} and R_{2d}, independently, is H or an amino-protecting group; R_{3d} is hydrogen, a hydroxy-protecting group or a residue of formula 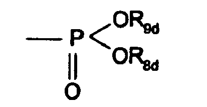 R_{4d} is C₁₋₄alkyl;
n_{d} is an integer of 1 to 6;
X_{d} is ethylene, vinylene, ethynylene, a group having a formula-D-CH₂- (wherein D is carbonyl, - CH(OH)-, O, S or N), aryl or aryl substituted by up to three substitutents selected from group a as defined hereinafter;
Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
R_{5d} is hydrogen, C₃₋₆cycloalkyl, aryl, heterocyclic group, C₃₋₆cycloalkyl substituted by up to three substituents selected from groups a and b, aryl substituted by up to three substituents selected from groups a and b, or heterocyclic group substituted by up to three substituents selected from groups a and b;
each of R_{6d} and R_{7d}, independently, is H or a substituent selected from group a;
each of R_{8d} and R_{9d}, independently, is H or C₁₋₄alkyl optionally substituted by halogen;
<group a > is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-C₁₋₄alkylamino, acylamino, cyano or nitro; and
<group b > is C₃₋₆cycloalkyl, aryl or heterocyclic group, each being optionally substituted by up to three substituents selected from group a;
with the proviso that when R_{5d} is hydrogen, Y_{d} is a either a single bond or linear C₁₋₁₀ alkylene, or a pharmacologically acceptable salt, ester or hydrate thereof;
- Compounds as disclosed in JP-14316985 (JP2002316985), e.g. a compound of formula VIII 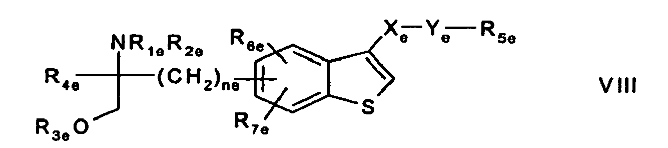
wherein R₁ₑ,R₂ₑ,R₃ₑ,R₄ₑ,Rₛₑ,R₆ₑ,R₇ₑ, nₑ, Xₑ and Yₑ are as disclosed in JP-14316985; or a pharmacologically acceptable salt, ester or hydrate thereof;
- Compounds as disclosed in WO 03/29184 and WO 03/29205, e.g. compounds of formula **IX** 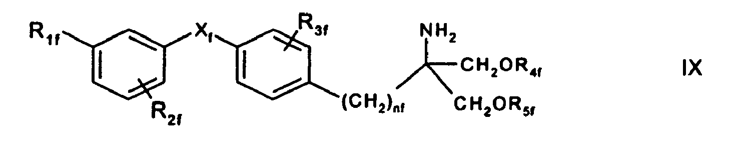
wherein X_{f} is O, S, SO or SO₂
R_{1f} is halogen, trihalomethyl, OH, C₁₋₇alkyl, C₁₋₄alkoxy, trifluoromethoxy, phenoxy, cyclohexylmethyloxy, pyridylmethoxy, cinnamyloxy, naphthylmethoxy, phenoxymethyl, CH₂-OH, CH₂-CH₂-OH, C₁₋₄alkylthio, C₁₋₄alkylsulfinyl, C₁₋₄alkylsulfonyl, benzylthio, acetyl, nitro or cyano, or phenyl, phenylC₁₋₄alkyl or phenyl-C₁₋₄alkoxy each phenyl group thereof being optionally substituted by halogen, CF₃, C₁₋₄alkyl or C₁₋₄alkoxy;
R_{2f} is H, halogen, trihalomethyl, C₁₋₄alkoxy, C₁₋₇alkyl, phenethyl or benzyloxy;
R_{3f} H, halogen, CF₃, OH, C₁₋₇alkyl, C₁₋₄alkoxy, benzyloxy or C₁₋₄alkoxymethyl;
each of R_{4f} and R_{5f}, independently is H or a residue of formula 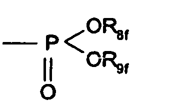 wherein each of R_{8f} and R_{9f}, independently, is H or C₁₋₄alkyl optionally substituted by halogen;and
n_{f} is an integer from 1 to 4;
e.g. 2-amino-2-[4-(3-benryloxyphenoxy)-2-chlorophenyl]ethyl-1,3-propane-diol, 2-amino-2-[4-(benzyloxyphenylthio)-2-chlorophenyl]ethyl-1,3-propane-diol, 2-amino-2-[4-(3-benzyloxyphenoxy)-2-chlorophenyl]propyl-1,3-propane-diol or 2-amino-2-[4-(benrytoxyphenylthio)-2- chlorophenyl]propyl-1,3-propane-diol,
or a pharmacological salt, solvate or hydrate thereof;
- Compounds as disclosed in WO03/062252A1, e.g. a compound of formula X 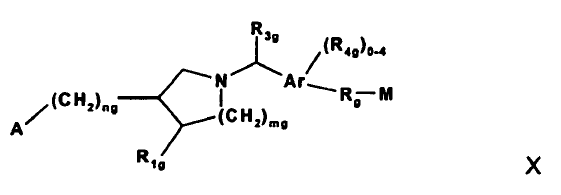
wherein
Ar is phenyl or naphthyl; each of m_{g} and n_{g} independently is 0 or 1; A is selected from COOH, PO₃H₂, PO₂H, SO₃H, PO(C₁₋₃alkyl)OH and 1*H*-tetrazol-5-yl; each of R_{1g} and R_{2g} independently is H, halogen, OH, COOH or C₁₋₄alkyl optionally substituted by halogen; R_{3g} is H or C₁₋₄alkyl optionally substituted by halogen or OH; each R_{4g} independently is halogen, or optionally halogen substituted C₁₋₄alkyl or C₁₋₃alkoxy; and each of R_{g} and M has one of the significances as indicated for B and C, respectively, in WO03/062252A1;
or a pharmacologically acceptable salt, solvate or hydrate thereof;
- Compounds as disclosed in WO 03/062248A2, e.g. a compound of formula XI 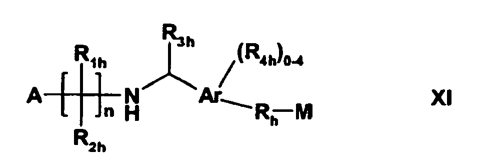
wherein Ar is phenyl or naphthyl; n is 2,3 or 4; A is COOH, 1*H-*tetrazol-5-yl, PO₃H₂, PO₂H₂, - SO₃H or PO(R₅ₕ)OH wherein R₅ₕ is selected from C₁₋₄alkyl, hydroxyC₁₋₄alkyl, phenyl, -CO-C₁-₃alkoxy and -CH(OH)-phenyl wherein said phenyl or phenyl moiety is optionally substituted; each of R₁ₕ and R₂ₕ independently is H, halogen, OH, COOH, or optionally halogeno substituted C₁₋₆alkyl or phenyl; R₃ₕ is H or C₁₋₄alkyl optionally substituted by halogen and/ OH; each R₄ₕ independently is halogeno, OH, COOH, C₁₋₄alkyl, S(O)_{0,1or2}C₁₋₃alkyl, C₁₋₃alkoxy, C₃₋₆cycloalkoxy, aryl or aralkoxy, wherein the alkyl portions may optionally be substituted by 1-3 halogens; and each of Rₕ and M has one of the significances as indicated for B and C, respectively, in WO03/062248A2;
- Compounds as disclosed in WO 04/026817A, e.g. compounds of formula XII 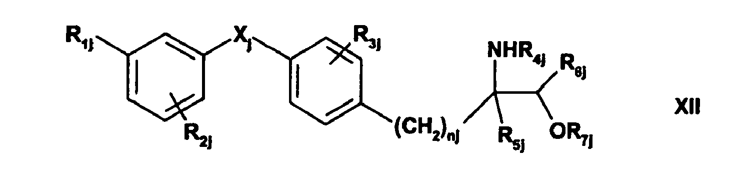
wherein
R₁ⱼ is halogen, trihalomethyl, C₁₋₄alkyl, C₁₋₄alkoxy, C₁₋₄alkylthio, C₁₋₄alkylsulifinyl, C₁₋₄alkylsulfonyl, aralkyl, optionally substituted phenoxy or aralkyloxy, R₂ⱼ is H, halogen, trihalomethyl, C₁₋₄alkyl, C₁₋₄alkoxy, aralkyl or aralkyloxy, R₃ⱼ is H, halogen, CF₃, C₁₋₄alkyl, C₁₋₄alkoxy, C₁₋₄alkylthio or benzyloxy, R₄ⱼ is H, C₁₋₄alkyl, phenyl, optionally substituted benzyl or benzoyl, or lower aliphatic C₁₋₅acyl, R₅ⱼ is H, monohalomethyl, C₁₋₄alkyl, C₁₋₄alkoxymethyl, C₁₋₄alkylthiomethyl, hydroxyethyl, hydroxypropyl, phenyl, aralkyl, C₂₋₄alkenyl or -alkynyl, each of R₆ⱼ and R₇ⱼ, independently, is H or C₁₋₄alkyl, Xⱼ is O, S, SO or SO₂ and nⱼ is an integer of 1 to 4, e.g. 2-amino-4-[4-(3-benzyloxyphenylthio)-2-chlorophenyl]-2-methylbutane-1-ol or 2-amino-4-[4-(3-benzy[oxyphenylthio)-2-chlorophenyl]-2-ethylbutane-1-ol;
- Compounds as disclosed in WO 04/103306A, WO 05/000833, WO 05/103309 or WO 05/113330, e.g. compounds of formula XIIIa or XIIIb 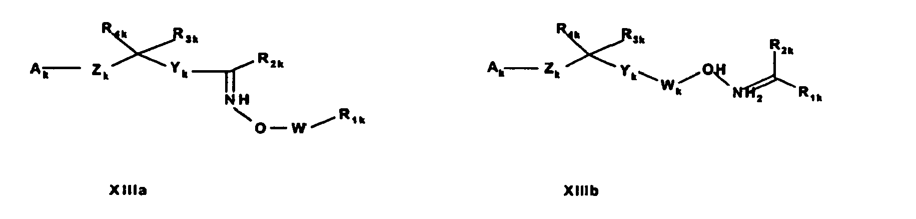
wherein
Aₖ is COOR₅ₖ, OPO(OR₅ₖ)₂, PO(OR₅ₖ)₂, SO₂OR₅ₖ, POR₅ₖOR₅ₖ or 1*H-*tetrazol-5-yl, R₅ₖ being H or C₁₋₆alkyl;
Wₖ is a bond, C₁₋₃alkylene or C₂₋₃alkenylene;
Yₖ is C₆₋₁₀aryl or C₃₋₉heteroaryl, optionally substituted by 1 to 3 radicals selected from halogene, OH, NO₂, C₁₋₆alkyl, C₁₋₆alky; halo-substituted C₁₋₆alkyl and halo-substituted C₁₋₆ₐlkoxy;
Zₖ is a heterocyclic group as indicated in WO 04/103306A, e.g. azetidine;
R₁ₖ is C₆₋₁₀aryl or C₃₋₉heteroaryl, optionally substituted by C₁₋₆alkyl, C₆₋₁₀aryl, C₆₋₁₀arylC₁₋₄alkyl, C₃₋₉heteroaryl, C₃₋₉heteroarylC₁₋₄alkyl, C₃₋₈cycloalkyl, C₃₋₈cycloalkylC₁₋₄alkyl, C₃₋₈heterocycloalkyl or C₃₋₈heterocycloalkylC₁₋₄alkyl; wherein any aryl, heteroaryl, cycloalkyl or heterocycloalkyl of R₁ₖ may be substituted by 1 to 5 groups selected from halogen, C₁₋₆alkyl, C₁₋₆alkoxy and halo substituted-C₁₋₆alkyl or -C₁₋₆alkoxy;
R₂ₖ is H, C₁₋₆alkyl, halo substituted C₁₋₆alkyl, C₂₋₆alkenyl or C₂₋₆alkynyl: and
each of R₃ₖ or R₄ₖ, independently, is H, halogen, OH, C₁₋₆alkyl, C₁₋₆alkoxy or halo substituted C₁₋₆alkyl or C₁₋₆alkoxy;
and the N-oxide derivatives thereof or prodrugs thereof,
or a pharmacologically acceptable salt, solvate or hydrate thereof.

According to a further embodiment of the invention, a S1P receptor agonist or modulator for use in the invention may also be a selective S1P1 receptor, e.g. a compound which possesses a selectivity for the S1P1 receptor over the S1P3 receptor of at least 20 fold, e.g. 100, 500, 1000 or 2000 fold, as measured by the ratio of EC₅₀ for the S1P1 receptor to the EC₅₀ for the S1P3 receptor as evaluated in a ³⁵S-GTPγS binding assay, said compound having an EC₅₀ for binding to the S1P1 receptor of 100 nM or less as evaluated by the ³⁵S-GTPγS binding assay. Representative S1P1 receptor agonists or modulators are e.g. the compounds listed in WO 03/061567, the contents of which being incorporated herein by reference, for instance a compound of formula XIV or XV

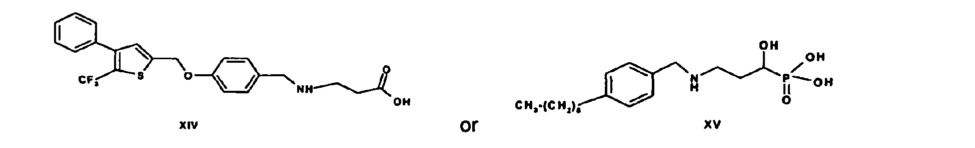

When the compounds of formulae I to XV have one or more asymmetric centers in the molecule, the present invention is to be understood as embracing the various optical isomers, as well as racemates, diastereoisomers and mixtures thereof are embraced. Compounds of formula III or IVb, when the carbon atom bearing the amino group is asymmetric, have preferably the R-configuration at this carbon atom.

The compounds of formulae I to XV may exist in free or salt form. Examples of pharmaceutically acceptable salts of the compounds of the formulae I to XV include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts of the combination of the present invention encompass hydrate and solvate forms.

Acyl as indicated above may be a residue R_{y}-CO- wherein R_{y} is C₁₋₆alkyl, C₃₋₈cycloalkyl, phenyl or phenyl-C₁₋₄alkyl. Unless otherwise stated, alkyl, alkoxy, alkenyl or alkynyl may be straight or branched.

Aryl may be phenyl or naphthyl, preferably phenyl.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

In the above formula of VII "heterocyclic group" represents a 5- to 7 membered heterocyclic group having 1 to 3 heteroatoms selected from S, O and N. Examples of such heterocyclic groups include the heteroaryl groups indicated above, and heterocyclic compounds corresponding to partially or completely hydrogenated heteroaryl groups, e.g. furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl, pyrazinyl, tetrahydropyranyl, morpholinyl, thiomorpholinyl, pyrrolidinyl, pyrrolyl, imidazolidinyl, pyrazolidinyl, piperidinyl, piperazinyl, oxazolidinyl, isoxazolidinyl, thiazolidinyl or pyrazolidinyl. Preferred heterocyclic groups are 5-or 6-membered heteroaryl groups and the most preferred heteocyclic group is a morpholinyl, thiomorpholinyl or piperidinyl group.

A preferred compound of formula I is 2-amino-2-tetradecyl-1,3-propanediol. A particularly preferred S1P receptor agonist of formula I is FTY720, i.e. 2-amino-2-[2-(4-octylphenyl) ethyl]propane-1,3-diol in free form or in a pharmaceutically acceptable salt form (referred to hereinafter as Compound A), e.g. the hydrochloride, as shown:

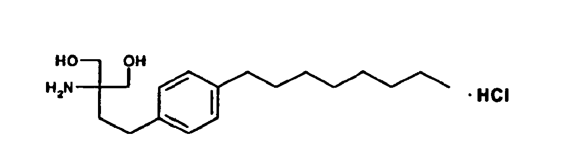

A preferred compound of formula II is the one wherein each of R'₂ to R'₅ is H and m is 4, i.e. 2-amino-2-{2-[4-(1-oxo-5-phenylpentyl)phenyl]ethyl}propane-1,3-diol, in free form or in pharmaceutically acceptable salt form (referred to hereinafter as Compound B), e.g the hydrochloride.

A preferred compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound C), e.g. the hydrochloride. The R-enantiomer is particularly preferred.

A preferred compound of formula IVa is the FTY720-phosphate (R₂ₐ is H, R₃ₐ is OH, Xₐ is O, R₁ₐ and R_{1b} are OH). A preferred compound of formula IVb is the Compound C-phosphate (R₂ₐ is H, R_{3b} is OH, Xₐ is O, R₁ₐ and R_{1b} are OH, Yₐ is O and R₄ₐ is heptyl). A preferred compound of formula V is Compound B-phosphate.

A preferred compound of formula V is phosphoric acid mono-[(R)-2-amino-2-methyl-4-(4-pentyloxy-phenyl)-butyl]ester.

A preferred compound of formula VIII is (2R)-2-amino-4-[3-(4-cyclohexyloxybutyl)-benzo[b]thien-6-yl]-2-methylbutan-1-ol.

A preferred compound of formula XIIIa is e.g. 1-{4-[1-(4-cyclohexyl-3-trifluoromethyl-benzyloxyimino)-ethyl]-2-ethyl-benzyl}-azetidine-3-carboxylic acid, or a prodrug thereof.

It has now been found that S1 P receptor modulators or agonists have a beneficial effect on Hepatitis C infections or Hepatitis C Virus (HCV)-induced disorders.

In a series of further specific or alternative embodiments, the present invention provides:
1.1 A method for preventing or treating HCV infections or HCV induced disorders in a subject in need thereof, comprising administering to said subject a therapeutically effective amount of an S1P receptor modulator or agonist, e.g. a compound of formulae VII to XV. HCV induced disorders may be e.g. liver cytoxicity, e.g. liver cell necrosis, or abnormal liver functions.
1.2 A method for inhibiting HCV replication in a medium, comprising applying to this medium an effective amount of an S1 P receptor modulator or agonist, e.g. a compound of formulae I to XV.
1.3 A method for inhibiting HCV replication in a subject in need thereof, comprising administering to this subject a therapeutically effective amount of an S1P receptor modulator or agonist, e.g. a compound of formulae I to XV.
1.4 A method for inhibiting HCV replication in a subject in need thereof having immunosuppression therapy, comprising administering to this subject a therapeutically effective amount of an S1P receptor modulator or agonist, e.g. a compound of formulae I to XV.
   By subject having immunosuppression therapy is meant a subject receiving a treatment with one or more immunosuppressants in order to prevent or lower the immune response of its body against, e.g. a cell, tissue or organ allo- or xeno-transplant (e.g. heart, lung, kidney, liver, bowel, pancreas, islet cells, stem cells, skin or corneal transplant or graft-versus-host diseases) in a graft recipient, or auto-antigens in a subject suffering from an autoimmune disease or disorder, e.g. multiple sclerosis, psoriasis, asthma, lupus nephritis, rheumatoid arthritis or inflammatory bowel diseases.
1.5 A method for preventing the recurrence of HCV infection or HCV induced disorders in a subject in need thereof having immunosuppression therapy, comprising administering to said recipient a therapeutically effective amount of an S1 P receptor modulator or agonist.
   A subject having immunosuppression therapy may be as defined above. A preferred method is for preventing the recurrence of HCV infection or HCV induced disorders in a transplant recipient.
1.6 A method for inhibiting, reducing or preventing hepatitis C viral cytotoxicity on liver cells in a subject in need thereof, comprising administering to said recipient a therapeutically effective amount of an S1P receptor modulator or agonist, e.g. a compound of formulae I to XV.
2. Use of an S1P receptor modulator or agonist in the preparation of a pharmaceutical composition for use in any method as defined above.
3. A pharmaceutical composition for use in any method as defined above, comprising an S1P receptor modulator or agonist together with one or more pharmaceutically acceptable diluents or carriers therefor.

The S1P receptor modulators or agonists of the invention may be administered as the sole ingredient or together with other drugs, e.g. a drug which has anti-HCV activities, e.g.
(1) Interferons.
   (a) Intron-A®, interferon alfa-2b (Schering Corporation, Kenilworth, NJ);
   (b) PEG-Intron®, peginteferon alfa-2b (Schering Corporation, Kenilworth, NJ);
   (c) Roferon®, recombinant interferon alfa-2a (Hoffmann-La Roche, Nutley, NJ);
   (d) Pegasys®, peginterferon alfa-2a (Hoffmann-La Roche, Nutley, NJ);
   (e) Berefor®, interferon alfa 2 available (Boehringer Ingelheim Pharmaceutical, Inc., Ridgefield, CT);
   (f) Sumiferon®, a purified blend of natural alpha interferons (Sumitomo, Japan)
   (g) Wellferon®, lymphoblastoid interferon alpha n1 (GtaxoSmithKline);
   (h) Infergen®, consensus alpha interferon (InterMune Pharmaceuticals, Inc., Brisbane, CA);
   (i) Alferon®, a mixture of natural alpha interferons (Interferon Sciences, and Purdue Frederick Co., CT);
   (j) Viraferon®;
   (k) Consensus alpha interferon from Amgen, Inc., Newbury Park, CA,
   (m) Other forms of interferon include: interferon beta, gamma, tau and omega, such as Rebif ( Interferon beta 1 a) by Serono, Omniferon (natural interferon) by Viragen, REBIF (interferon beta-1a) by Ares-Serono, Omega Interferon by BioMedicines or Intarcia; oral Interferon Alpha by Amarillo Biosciences; an interferon conjugated to a water soluble polymer or to a human albumin, e.g., Albuferon (Human Genome Sceince), Alfaferone (Alfa Wassermann SpA, ), interferon alfacon-1 (WO05067454, Amgen), AERX (an inhaled interferon alpha-2b by Aradigm), Albuferon (Aventis), controlled release interferon alpha (Biolex/Octoplus), controlled release interferon aipha-2b (Flamel Technologies), Actimmune (Genentech), Interferon alpha-n1 (Glaxo Wellcome), hydroxocobalamin (Transition Therapeutics);
   (n) Conjugates of interferon to a water-soluble polymer include especially conjugates to polyalkylene oxide homopolymers such as polyethylene glocol (PEG) or polypropylene glycols, polyoxyethylenated polyols, copolymers thereof and block copolymers thereof. As an alternative to polyalkylene oxid-based polymers, effectively non-antigenic materials such as dextran, polyvinyl pyrrolidones, polyacrylamides, polyvinyl alcohols, carbohydrate-based polymers and the like can be used. Since the polymeric modification reduces antigenic response, the foreign interferon need not be completely autologous. Interferon used to prepare polymer conjugates may be prepared from a mammalian extract, such as human, ruminant or bovine interferon, or recombinantly produced. Preferred are conjugates of interferon to polyethylene glycol, also known as pegylated interferons.
   Examples of conjugates of interferon are e.g. pegylated alfa-interferons, for example pegylated interferon-alfa-2a, pegylated interferon-alfa-2b; Pegylated consensus interferon or pegylated purified interferon-alfa product. Pegylated interferon-alfa-2a is described e.g. in European Patent 593,868 and commercially available e. g. under the tradename PEGASYS^{®} (Hoffmann-La Roche). Pegylated interferon-alfa-2b is described, e.g. in European Patent 975,369 and commercially available e.g. under the tradename PEG-INTRON A^{®} (Schering Plough). Pegylated consensus interferon is described in WO 96/11953.
(2) Antiviral agents.
   Antiviral agents may be compounds or biologicals that are effective to inhibit the formation and/or replication of a virus in a mammal. This includes agents that interfere with either host or viral mechanisms necessary for the formation and/or replication of a virus in a mammal. Antiviral agents include, for example, Ribavirin (1-beta-D-ribofuranosyl-1H-1,2,4-triazole-3-carboxamide) from Valeant Pharmaceuticals, Inc., Costa Mesa, CA); Rebetol® from Schering Corporation, Kenilworth, NJ, and Copegus® from Hoffmann-La Roche, Nutley, NJ; and new ribavirin analogues in development such as LEVOVIRIN and VIRAMIDINE by Valeant, amantadine, VX-497 (MERIMEPODIB, Vertex Pharmaceuticals), VX-498 (Vertex Pharmaceuticals), Ceplene (a histamine dichloride, by Maxim), XTL-001 and XTL-002 (XTL Biopharmaceuticals), Viramidine (WO00160379), human leukocyte-derived alpha interferon (ViraNative AB); lamivudine; the compounds disclosed in US patent no. 6,812,219 and WO 2004/002422 A2 (the disclosures of which are incorporated herein by reference in their entireties); an inhibitor of the HCV or other Flaviviridae virus encoded factors like the NS3/4A protease;
(3) Thiazolidine derivatives which show relevant inhibition in a reverse-phase HPLC assay with an NS3/4A fusion protein and NS5A/5B substrate (Sudo K. et al., Antiviral Research, 1996, 32, 9-18), especially compound RD-1-6250, possessing a fused cinnamoyl moiety substituted with a long alkyl chain, RD4 6205 and RD4 6193;
(4) Thiazolidines and benzanilides identified in Kakiuchi N. et al. J. FEBS Letters 421, 217-220; Takeshita N. et al. Analytical Biochemistry, 1997, 247, 242-246;
(5) A phenanthrenequinone possessing activity against protease in a SDS-PAGE and autoradiography assay isolated from the fermentation culture broth of Streptomyces sp., Sch 68631 (Chu M. et al., Tetrahedron Letters, 1996, 37, 7229-7232), and Sch 351633, isolated from the fungus *Penicillium griseofulvum,* which demonstrates activity in a scintillation proximity assay (Chu M. et al, Bioorganic and Medicinal Chemistry Letters 9, 1949-1952);
(6) Protease inhibitors

Examples include substrate-based NS3 protease inhibitors (Attwood et al., *Antiviral peptide derivatives,* PCT WO 98/22496, 1998; Attwood et al., Antiviral Chemistry and Chemotherapy 1999, 10, 259-273; Attwood et al, *Preparation and use of amino acid derivatives* as *anti-viral* agents, German Patent Pub. DE 19914474; Tung et al. *Inhibitors of serine proteases, particularly hepatitis C virus NS3 protease*; PCT WO 98/17679), including alphaketoamides and hydrazinoureas, and inhibitors that terminate in an electrophile such as a boronic acid or phosphonate (Llinas-Brunet et al. *Hepatitis* C *inhibitor peptide analogues,* PCT WO 99/07734) are being investigated.

Non-substrate-based NS3 protease inhibitors such as 2,4,6-trihydroxy-3-nitro-benzamide derivatives (Sudo K. et al., Biochemiscal and Biophysical Research Communications, 1997, 238 643-647; Sudo K. et al. Antiviral Chemistry and Chemotherapy, 1998, 9, 186), including RD3-4082 and RD3-4078, the former substituted on the amide with a 14 carbon chain and the latter processing a para-phenoxyphenyl group are also being investigated.

Sch 68631, a phenanthrenequinone, is an HCV protease inhibitor (Chu M et al., Tetrahedron Letters 37:7229-7232, 1996). In another example by the same authors, Sch 351633, isolated from the fungus *Penicillium grieofulvum,* was identified as a protease inhibitor (Chu M. et al., Bioorganic and Medicinal Chemistry Letters 9:1949-1952). Nanomolar potency against the HCV NS3 protease enzyme has been achieved by the design of selective inhibitors based on the macromolecule eglin c. Eglin c, isolated from leech, is a potent inhibitor of several serine proteases such as S. griseus proteases A and B, ∀-chymotrypsin, chymase and subtilisin. Qasim M.A. et al., Biochemistry 36:1598-1607, 1997.

U.S. patents disclosing protease inhibitors for the treatment of HCV include, for example, U.S. Patent No. 6,004,933 to Spruce et al (incorporated herein by reference in its entirety) which discloses a class of cysteine protease inhibitors for inhibiting HCV endopeptidase 2; U.S. Patent No. 5,990,276 to Zhang et al.(incorporated herein by reference in its entirety) which discloses synthetic inhibitors of hepatitis C virus NS3 protease; U.S. Patent No. 5,538,865 to Reyes et al.(incorporated herein by reference in its entirety). Peptides as NS3 serine protease inhibitors of HCV are disclosed in WO 02/008251 to Corvas International, Inc., and WO 02/08187 and WO 02/008256 to Schering Corporation (incorporated herein by reference in their entireties). HCV inhibitor tripeptides are disclosed in U.S. Patent Nos. 6,534,523, 6,410,531 and 6,420,380 to Boehringer Ingelheim and WO 02/060926 to Bristol Myers Squibb (incorporated herein by reference in their entireties). Diaryl peptides as NS3 serine protease inhibitors of HCV are disclosed in WO 02/48172 to Schering Corporation (incorporated herein by reference).

Imidazoleidinones as NS3 serine protease inhibitors of HCV are disclosed in WO 02/18198 to Schering Corporation and WO 02/48157 to Bristol Myers Squibb (incorporated herein by reference in their entireties). WO 98/17679 to Vertex Pharmaceuticals and WO 02/48116 to Bristol Myers Squibb also disclose HCV protease inhibitors (incorporated herein by reference in their entireties).

HCV NS3-4A serine protease inhibitors including BILN 2061 by Boehringer Ingelheim, compounds described in WO 99/07733, WO 99/07734, WO 00/09558, WO 00/09543, WO 00/59929 or WO 02/060926, and the Vertex/Eli Lilly pre-development candidate identified as VX-950 or LY-570310 in WO 02/060926, SCH 6/7 by Schering-Plough, and other compounds currently in preclinical development;

Substrate-based NS3 protease inhibitors, including alphaketoamides and hydrazinoureas, and inhibitors that terminate in an elecrophile such as a boronic acid or phosphonate; Non-substrate-based NS3 protease inhibitors such as 2,4,6-trihydroxy-3-nitro-benzamide derivatives including RD3-4082 and RD3-4078, the former substituted on the amide with a 14 carbon chain and the latter processing a para-phenoxyphenyl group; and Sch503034 (202005087721, ScheringPlough).

Sch 351633, isolated from the fungus *Penicillium griseofulvum* was identified as a protease inhibitor. Eglin c, isolated from leech is a potent inhibitor of several serine proteases such as S. griseus proteases A and B, a-chymotrypsin, chymase and subtilisin.

US patent no. 6004933 (incorporated herein by reference in its entirety) discloses a class of cysteine protease inhibitors from inhibiting HCV endopeptidase 2; synthetic inhibitors of HCV NS3 protease (pat), HCV inhibitor tripeptides (pat), diaryl peptides such as NS3 serine protease inhibitors of HCV (pat), Imidazolidindiones as NS3 serine protease inhibitors of HCV (pat).

Thiazolidine derivatives which show relevant inhibition in a reverse-phase HPLC assay with an NS3/4A fusion protein and NS5A/5B substrate especially compound RD-16250 possessing a fused cinnamoyl moiety substituted with a long alkyl chain, RD4 6205 and RD4 6193.
(7) Nucleoside or non-nucleoside inhibitors of HCV NS5B RNA-dependent RNA polymerase, such as 2'-C-methyl-3'-O-L-valine ester ribofuranosyl cytidine (Idenix Pharmaceuticals) as disclosed in WO 2004/002422 A2 (incorporated herein by reference in its entirety). Idenix Pharmaceuticals also discloses the use of branched nucleosides in the treatment of flaviviruses (including HCV) and pestiviruses WO 01/90121 and WO 01/92282 (incorporated herein by reference in their entireties). Specifically, a method for the treatment of hepatitis C infection (and flaviviruses and pestiviruses) in humans and other host animals is disdosed in these publications which includes administering an effective amount of a biologically active 1', 2', 3' or 4'-branced B-D or B-L nucleosides or a pharmaceutically acceptable salt or prodrug thereof, administered either alone or in combination with another antiviral agent, optionally in a pharmaceutically acceptable carrier. Other nucleoside analogs include those in WO 02/057287 A2, WO 02/057425 A2, WO 01/90121 and US 6812219.

Other non-nucleoside polymerase inhibitors include those compounds disclosed in U.S. application Ser. No. 10/198,680, U.S. application Ser. No. 10/198,384, U.S. application Ser. No. 10/198,259, (all from Boehringer Engelheim); WO 02/100846 A1 and WO 02/100851 A2 (both Shire), WO 01/85172 A1 and WO 02/098424 A1 (both GSK), WO 00/06529 and WO 02/06246 A1 (both Merck), WO 01/47883 and WO 03/000254 (both Japan Tobacco) and EP 1 256 628 A2 (Agouron)

Other patent applications disclosing the use of certain nucleoside analogs to treat hepatitis C virus include: PCTCA00/01316 (WO 01/32153; filed November 3, 2000) and PCT/CA01/00197 (WO 01/60315; filed February 19, 2001) filed by BioChem Pharma, Inc., (now Shire Biochem, Inc.); PCT/US02/01531 (WO 02/057425; filed January 18, 2002) and PCT/US02/03086 (WO 02/057287; filed January 18, 2002) filed by Merck & Co., Inc., PCT/EP01/09633 (WO 02/18404; published August 21, 2001) filed by Roche, and PCT Publication Nos. WO 01/79246 (filed April 13, 2001), WO 02/32920 (filed October 18, 2001) and WO 02/48165 by Pharmasset, Ltd. (the disclosures of which are incorporated herein by reference in their entireties)

PCT Publication No. WO 99/43691 to Emory University (incorporated herein by reference in its entirety), entitled "2'-Fluoronucleosides" discloses the use of certain 2'-fluoronucleosides to treat HCV.

Eldrup et al. (Oral Session V, Hepatitis C Virus, Flaviviridae; 16th International Conference on Antiviral Research (April 27, 2003, Savannah, GA)) described the structure activity relationship of 2'-modified nucleosides for inhibition of HCV.

Bhat et al. (Oral Session V, Hepatitis C Virus, Flaviviridae, 2003 (Oral Session V, Hepatitis C Virus, Flaviviridae; 16th International conference on Antiviral Research (April 27, 2003, Savannah, Ga); p A75) describes the synthesis and pharmacokinetic properties of nucleoside analogues as possible inhibitors of HCV RNA replication. The authors report that 2'-modified nucleosides demonstrate potent inhibitory activity in cell-based replicon assays.

Olsen et al. (Oral Session V, Hepatitis C Virus, Flaviviridae; 16th International Conference on Antiviral Research (April 27, 2003, Savannah, Ga)p A76) also described the effects of the 2'-modified nucleosides on HCV RNA replication.
(8) Nucleotide polymerase inhibitors and gliotoxin (Ferrari R. et al. Journal of Virology, 1999, 73, 1649-1654), and the natural product cerulenin (Lohmann V. et al. Virology, 1998, 249, 108-118),
(9) Helicase inhibitors.

Heterocyclic substituted carboxamic inhibitors (Diana G.D. et al., U.S. 5,633,388) and piperidine derivatives (WO 97/36554); VP-50406 by ViroPhama and preclinical compounds from Vertex.
(10) Antisense.

Phosphorothioate oligodeoxynucleotides (S-ODN) complementary to sequence stretches in the 5' non-coding region (NCR) of the virus (Alt M. et al., Hepatology, 1995, 22, 707-717), or nucleotides 326-348 comprising the 3' end of the NCR and nucleotides 371-388 located in the core coding region of the HCV RNA (Alt M. et al., Archives of Virology, 1997, 142, 589-599; Galderisi U. et al., Journal of Cellular Physiology, 199, 181, 251-257); such as ISIS 14803 by Isis Pharm/Elan, antisense by Hybridon, antisense by AVI bioPharma, AVI-4065 (AVI BioPharma), or an antisense sequence complementary to any part of the HCV genome which increases effectiveness of therapy.
(11) Inhibitors of IRES-dependent translation (Ikeda N et al., *Agent for the prevention and treatment of hepatitis C*, Japanese Patent Pub. JP-08268890; Kai Y et al. *Prevention and treatment of viral diseases,* Japanese Patent Pub. JP-10101591); such as ISIS 14803 by Isis Pharm/Elan, IRES inhibitor by Anadys, IRES inhibitors by lmmusol, targeted RNA chemistry by PTC Therapeutics
(12) Ribozymes, such as nuclease-resistant ribozymes (Maccjak, D.J. et al., Hepatology 1999, 30, abstract 995) and those directed in U.S. Patent No. 6,043,077 to Barber et al., and U.S. Patent Nos. 5,869,253 and 5,610,054 to Draper et al.(incorporated herein by reference in their entireties) for example, HEPTAZYME by RPI
(13) Receptor agonists such as such as isatoribine (ANA975 and ANA245) nucleoside analogs which are TOLL-like receptor agonist (U.S. 5,041,426 and 4,880,784), CpG-10101 (TLR-9 agonist, Coley Pharmaceuticals)
(14) Substituted thioaryl urea derivatives such as 1-(4-pentytoxy-3-trifluoromethylphenyl)-3-(pyridine-3-carbonyl) thiourea, (U.S. Publication 2004/0138205, U.S. Ser. No. 10/716,175).
(15) An immune modulating agent such as an IMPDH inhibitor, mycophenolic acid, a salt or a prodrug thereof sodium mycophenolate or mycophenolate mofetil, or Merimebodib (VX-497); thymosin alpha-1 (Zadaxin, by SciClone); or a S1P receptor agonist, e.g. FTY720 or analogue thereof optionally phosphorylated.
(16) An anti-fibrotic agent, such as a N-phenyl-2-pyrimidine-amine derivative, imatinib (Gleevac), IP-501 by Indevus, and Interferon gamma 1b from InterMune
(17) Therapeutic vaccines by Intercell, Epimmune/Genecor, Merix, Tripep (Chron-VacC), immunotherapy (Therapore) by Avant, T cell therapy by CellExSys, monoclonal antibody XTL-002 by STL, ANA 246 and ANA 246 by Anadys, GI-5005 (Globelmmune Inc), lnnoVac-C (WO 9967285, Innogenetics), IC-41 (Intercell), interferon alfa-n3 (Interferon Sciences), Engerix B (SmithKline Beecham),
(18) Other miscellaneous compounds or preclinical compounds include Ursodiol (EP00269516, Axcan Pharma), HE-2000 (a DHEA analog, Colthurst Ltd), EHC-18 (an immunomodulator, Enzo biochem), histamine dihydrochloride (an H2 agonist, WO09104037, Estero-Anstalt), 1-amino-alkylcyclohexanes (U.S. Patent No. 6,034,134 to Gold et al.), Celgosivir (an alpha glucosidase, WO02089780, by Hoechst Mario Roussel), KPE-2003002 (a phytochemical derivative, Kemin Pharma Europe), KPE00001133 (a bicyclic carbohydrate derivative, Kemin Pharma Europe ), KRN7000 (JP-09315980, Kirin Brewery), Civacir (antibodies, Nabi Biopharmaceuticals), anti-phosphatidyl serine (University of Texas), NOV-205 (Novelos), Virostat (methylene blue, Oklahoma Medical Research Foundation), UT-231 B (alpha galactosidase inhibitors, Oxford University), PYN-17 (phytochemical derivatives, WO2005079823, Phynova Ltd), thymosin alpha (RegeneRx Biopharmaceuticals), propagermanium (Sanwa Kagaku Kenkyusho Co), compounds disclosed in Chen et al, Expert Opin. Ther. Patents 15(8):1027-1039 (2005), SCV-07 (immunomodulator, WO9933799), alkyl lipids (U.S. Pat. No. 5,922,757 to Chojkier et al.), vitamin E and other antitoxidants (U.S. Patent. No. 5,922,757 to Chojkier et al.), bile acids (U.S. Pat. No. 5,846,99964 to Ozeki et al.), N-(phosphonoacetl)-L-aspartic acid, (U.S. Pat. No. 5,830,905 to Diana et al.), benzenedicarboxamides (U.S. Pat. No. 5,633,388 to Diane et al.), polyadenylic acid derivatives (U.s. Pat. No. 5,496,546 to Wang et al.), 2'3'-dideoxyinosine (U.S. Pat. No. 5,026,687 to Yarchoan et al.), benzimidazoles (U.S. Pat. No. 5,891,874 to Colacino et al.), plant extracts (U.S. Pat. No. 5,837,257 to Tsai et al., U.S. Pat. No. 5,725,859 to Omer et al., and U.S. Pat. No. 6,056,961) and piperidines (U.S. Pat. No. 5,830,905 to Diana et al.); the disclosures of which are incorporated herein by reference in their entireties. Also, squalene, telbivudine, N-(phosphonoacetyl)-L-aspartic acid, benzenedicarboxamides, polyadenylic acid derivatives, glycosylation inhibitors, and nonspecific cytoprotective agents that block cell injury caused by the virus infection.
(19) Other compounds also currently in preclinical or clinical development for the treatment of HCV, include Interleukin-10 (Schering-Plough), SYMMETREL by Endo Labs Solvay, caspase inhibitor IDN-6556 by Idun Pharma, HCV/MF59 by Chiron, CIVACIR (A hepatitis C immune globulin by NABI), IDN-6556 by Idun Pharm, T67 (a beta-tubulin inhibitor, by Tularik), a therapeutic vaccine directed to E2 (by Innogenetics), FK788 (by Fujisawa Healthcare), IdB1016 (Siliphos, an oral silybin-phosphatidyl choline phytosome), fusion inhibitors (by Trimeris), Dication (by Immtech), hemopurifier (by Aethlon Medical), UT 231 B (by United Therapeutics), R803 (Rigel), JTK-003, JTK-002, and JTK-109 (all from Japan Tobacco), HCV-086 (ViroPharma/Wyeth), ISIS-14803 (ISIS Pharmaceuticals), GS-9132 (a polymerase inhibitor by Achillion Pharmaceuticals), HCV-793 (a polymerase inhibitor, ViroPharma), HepeX-C (antibodies, XTL Biopharmaceuticals), PSI-6130 (Pharmasset and Roche), R1626 (Roche) as well as other compounds currently in preclinical development which can easily be obtained by one of skill in the art by search clinical trial information and press release information from respective companies.

In accordance with the foregoing the present invention provides in a yet further aspect:
4. A pharmaceutical combination comprising a) a first agent which is an S1P receptor modulator or agonist, e.g. a compound of formulae I to XV, and b) a co-agent, e.g. a second drug agent as defined above.
5. A method as defined above comprising co-administration, e.g. concomitantly or in sequence, of a therapeutically effective amount of an S1 P receptor modulator or agonist, e.g. a compound of formulae I to XV, and a co-agent, e.g. a second drug agent as defined above.
6. A therapeutic combination, e.g. a kit, comprising a) an S1 P receptor modulator or agonist, e.g. a compound of formulae I to XV, and b) at least one second drug substance, said second drug substance being as defined above. Component a) and component b) may be used concomitantly or in sequence. The kit may comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

The administration of a pharmaceutical combination of the invention results in a beneficial effect, e.g. a synergistic therapeutic effect, compared to a monotherapy applying only one of its pharmaceutically active ingredients. A preferred synergistic combination is a combination of an S 1 P receptor modulator or agonist with pegylated alfa-interferon.

In each case where citations of patent applications are given above, the subject matter relating to the compounds is hereby incorporated into the present application by reference. Comprised are likewise the pharmaceutically acceptable salts thereof, the corresponding racemates, diastereoisomers, enantiomers, tautomers as well as the corresponding crystal modifications of above disclosed compounds where present, e.g. solvates, hydrates and polymorphs, which are disclosed therein. The compounds used as active ingredients in the combinations of the invention can be prepared and administered as described in the cited documents, respectively. Also within the scope of this invention is the combination of more than two separate active ingredients as set forth above, i.e. a pharmaceutical combination within the scope of this invention could include three active ingredients or more.

Utility of an S1P receptor modulator or agonist in treating diseases and conditions as hereinabove specified may be demonstrated in standard animal or clinical tests, e.g. in accordance with the methods described hereinafter.

### A. Assays

A1. Luciferase-based HCV replicon assay:
   The HCV replicon cell line, Huh-Luc/neo-ET, is obtained from ReBlikon GmbH. The cells are cultured in Dulbecco's modified Eagle's medium (DMEM, Gibco), containing 10% heat-inactivated fetal bovine serum (FBS, Gibco), 2 mM L-glutamine, 1x nonessential amino acids (Gibco), and 0.25 mg/ml G418 (Invitrogen, Carlsbad, CA). In this cell line, the expression of luciferase is under the control of HCV RNA replication and translation, therefore the antiviral activity can be determined by measuring the reduction of luciferase activity in the cells. HCV replicon cells (Huh-Luc/neo-ET) are treated with a compound to be tested serially diluted in DMEM supplemented with 10% FBS and 0.5% DMSO for 48 h. Then the luciferase activity is determined using BrightGlo reagent (Promega) and a LMaxll plate reader (Molecular Probe). To monitor the cytotoxic effect of the compound to be tested, the viability of the replicon cells following 48 h of compound treatment is determined using a tetrazolium compound (MTS)-based assay (CellTiter 96® AQueous One Solution Cell Proliferation Assay, Promega, Madison, WI). CC50 is the concentration of the compound at which the cell viability is reduced by 50%.
A.2 The compound to be tested is administered orally for 5 days to a group of mice infected by inoculation with Hepatitis C virus. A control group of mice receives distilled water. Five days later the liver is harvested and liver cell necrosis is scored according to known methods and compared with the control animals.

### B. Clinical Trial

A person suffering from hepatitis C infection, in particular chronic HCV infection, may exhibit one or more of the following signs or symptoms: (a) elevated ALT, (b) positive test for anti-HCV antibodies, (c) presence of HCV as demonstrated by a positive test for HCV-RNA, (d) clinical stigmata of chronic liver disease, (e) hepatocellular damage. Such criteria may not only be used to diagnose hepatitis C, but can be used to evaluate a patient's response to drug treatment.

Elevated serum alanine aminotransferase (ALT) and aspartate aminotransferase (AST) are known to occur in uncontrolled hepatitis C, and a complete response to treatment is generally defined as the normalization of these serum enzymes, particularly ALT (Davis et al., 1989, New Eng. J. Med. 321:1501-1506). ALT is an enzyme released when liver cells are destroyed and is symptomatic of HCV infection. Interferon causes synthesis of the enzyme 2',5'-oligoadenylate synthetase (2'5'OAS), which in turn, results in the degradation of the viral mRNA. Houglum, 1983, Clinical Pharmacology 2:20-28. Increases in serum levels of the 2'5'OAS coincide with decrease in ALT levels.

In order to follow the course of HCV replication in subjects in response to drug treatment, HCV RNA may be measured in serum samples by, for example, a nested polymerase chain reaction assay that uses two sets of primers derived from the N53 and N54 non-structural gene regions of the HCV genome. Farci et al., 1991, New Eng. J. Med. 325:98-104. Ulrich et al., 1990, J. Clin. Invest., 86:1609-1614.

Histological examination of liver biopsy samples may be used as a second criteria for evaluation. See, e.g., Knodell et al., 1981, Hepatology 1:431-435, whose Histological Activity Index (portal inflammation, piecemeal or bridging necrosis, Iobular injury and fibrosis) provides a scoring method for disease activity. The compounds tested may be e.g. Compound A or a compound of formula IX, XII or XIIIa or XIIIb.

### C. Combined Treatment

Suitable clinical studies are, for example, open label, dose escalation studies in patients. Such studies prove in particular the synergism of the active ingredients of the combination of the invention. The beneficial effects can be determined directly through the results of these studies which are known as such to a person skilled in the art. Such studies are, in particular, suitable to compare the effects of a monotherapy using the active ingredients and a combination of the invention. Preferably, the dose of agent (a) is escalated until the Maximum Tolerated Dosage is reached, and agent (b) is administered with a fixed dose. Alternatively, the agent (a) is administered in a fixed dose and the dose of agent (b) is escalated. Each patient receives doses of the agent (a) either daily or intermittent. The efficacy of the treatment can be determined in such studies, e.g., after 12, 18 or 24 weeks by evaluation of symptom scores every 6 weeks.

Alternatively, a placebo-controlled, double blind study can be used in order to prove the benefits of the combination of the invention mentioned herein, e.g. in transplantation of an organ, tissue or cells, e.g. Langerhans islet cells.

More specifically, clinical trials B1 to B4, as described hereafter, can be used in order to prove the benefits of a combination of an S1P receptor agonist and a pegylated interferon.

B1: A pilot trial measuring the viral load (HCV-RNA), the serum alanine aminotransferases (ALT) and the aspartate aminotransferases (AST) and standard safety parameters (e.g. other liver function tests, blood cell count and biochemistry) at H0, H8, H12, H24, D2, D5, D7, D14, D21, D28 (where H is hour, D is day, 0 is time of first administration of treatment) in four groups of patients:
Group 1: Compound A given in 2 divided doses, e.g. 1 to 30 mg/day
Group 2: Compound A given in 4 divided doses, e.g. 1 to 30 mg/day
Group 3: Compound A given in 2 divided doses + pegylated interferon;
Group 4: Compound A given in 4 divided doses + pegylated interferon.

All patients will be patients who are infected by hepatitis C virus and present with abnormal liver function tests, especially abnormal ALT's. All of them will be "naïve" patients, i.e. none of them will already have received any kind of anti-viral treatment against hepatitis C (interferon and/or ribavirin).

B2: A similar trial as B1 performed in patients who have failed to respond to a treatment combining interferon or pegylated interferon and ribavirin.

B3: A trial comparing the combination of pegylated interferon and Compound A to the standard combination of pegylated interferon and ribavirin. The dosage of Compound A will be defined as per the results of the pilot trials. The assessment criteria will be a sustained virological response 48 weeks after the end of a 24 (HCV genotype 2-3) or 48 (HCV genotype 1) week treatment. The trial will be conducted in "naïve" patients.

B4: A trial conducted in patients who failed to respond to a treatment combining interferon or pegylated interferon and ribavirin. This trial will compare the rate of virological response obtained with Compound A alone and with the combination of interferon or pegylated interferon and Compound A. The dosage of Compound A will be defined as per the results of the pilot trials.

Above procedure may be repeated using a compound of formula IX, XII, XIIIa or XIIIb.

The administration of a pharmaceutical combination of the invention results not only in a beneficial effect, e.g. a synergistic therapeutic effect, e.g. with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically active ingredients used in the combination of the invention.

A further benefit is that lower doses of the active ingredients of the combination of the invention can be used, for example, that the dosages need not only often be smaller but are also applied less frequently, which may diminish the incidence or severity of side-effects. This is in accordance with the desires and requirements of the patients to be treated.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

It is one objective of this invention to provide a pharmaceutical composition comprising a quantity, which is jointly therapeutically effective against graft rejection or autoimmune diseases or disorders associated therewith comprising a combination of the invention. In this composition, agent a) and agent (b) may be administered together, one after the other or separately in one combined unit dosage form or in two separate unit dosage forms. The unit dosage form may also be a fixed combination.

The pharmaceutical compositions for separate administration of agent a) and agent b) or for the administration in a fixed combination, i.e. a single galenical composition comprising at least two combination partners a) and b), according to the invention may be prepared in a manner known per se and are those suitable for enteral, such as oral or rectal, and parenteral administration to mammals (warm-blooded animals), including humans, comprising a therapeutically effective amount of at least one pharmacologically active combination partner alone, e.g. as indicated above, or in combination with one or more pharmaceutically acceptable carriers or diluents, especially suitable for enteral or parenteral application.

Suitable pharmaceutical compositions contain, for example, from about 0.1 % to about 99.9%, preferably from about 1 % to about 60 %, of the active ingredient(s). Pharmaceutical preparations for the combination therapy for enteral or parenteral administration are, for example, those in unit dosage forms, such as sugar-coated tablets, tablets, capsules or suppositories, or ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partner of the combination of the invention may be administered simultaneously or sequentially and in any order, and the components may be administered separately or as a fixed combination. For example, the method of preventing or treating graft rejection or autoimmune diseases according to the invention may comprise (i) administration of the first agent a) in free or pharmaceutically acceptable salt form and (ii) administration of an agent b) in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual combination partners of the combination of the invention may be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimens of simultaneous or alternating treatment and the term "administering" is to be interpreted accordingly.

The effective dosage of each of the combination partners employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A physician, clinician or veterinarian of ordinary skill can readily determine and prescribe the effective amount of the single active ingredients required to alleviate, counter or arrest the progress of the condition. Optimal precision in achieving concentration of the active ingredients within the range that yields efficacy without toxicity requires a regimen based on the kinetics of the active ingredients' availability to target sites.

Daily dosages for agent a) or b) or will, of course, vary depending on a variety of factors, for example the compound chosen, the particular condition to be treated and the desired effect. In general, however, satisfactory results are achieved on administration of agent a) at daily dosage rates of the order of ca. 0.03 to 5 mg/kg per day, particularly 0.1 to 5 mg/kg per day, e.g. 0.1 to 2.5 mg/kg per day, as a single dose or in divided doses. The S1 P receptor modulator or agonist, e.g. a compound of formulae I to XV, may be administered by any conventional route, in particular enterally, e.g. orally, e.g. in the form of tablets, capsules, drink solutions or parenterally, e.g. in the form of injectable solutions or suspensions. Suitable unit dosage forms for oral administration comprise from ca. 0.02 to 50 mg active ingredient, usually 0.1 to 30 mg, e.g. Compound A or B, together with one or more pharmaceutically acceptable diluents or carriers therefor.

Agent b) may be administered to a human in a daily dosage range of 0.5 to 1000 mg. Suitable unit dosage forms for oral administration comprise from ca. 0.1 to 500 mg active ingredient, together with one or more pharmaceutically acceptable diluents or carriers therefore. For example, lamivudine may be administered at a daily dosage of 100mg. Ribavirin may be administered at a daily dose of 1.0 to 1.5 g. Mycophenolate sodium may be administered at a daily dose of from 180 mg to 720 mg. The pegylated interferon may be administered parenterally one to three times per week, preferably once a week, at a total weekly dose ranging from 2 to 10 million IU, more preferable 5 to 10 million IU, most preferable 8 to 10 million IU. Because of the diverse types of co-agent that may be used, the amounts can vary greatly, e.g., 0.0001 to 5,000 mg/kg per day.

The administration of a pharmaceutical combination of the invention results not only in a beneficial effect, e.g. a synergistic therapeutic effect, e.g. with regard to inhibiting graft rejection in transplanted patients or slowing down or arresting autoimmune disorders, but also in further surprising beneficial effects, e.g. less side-effects, an improved quality of life or a decreased morbidity, compared to a monotherapy applying only one of the pharmaceutically active ingredients used in the combination of the invention.

A further benefit is that lower doses of the active ingredients of the combination of the invention can be used, for example, that the dosages need not only often be smaller but are also applied less frequently, or can be used in order to diminish the incidence of side-effects. This is in accordance with the desires and requirements of the patients to be treated.

## Claims

1. An S1P receptor modulator or agonist for use in a method for preventing the recurrence of HCV infection or HCV induced disorders in a subject in need thereof having immunosuppression therapy.

2. An S1P receptor modulator or agonist for use in a method for preventing or treating HCV infections or HCV induced disorders in a subject in need thereof, wherein the HCV induced disorder is liver cell necrosis or abnormal liver functions.

3. An S1P receptor modulator or agonist according to claim 1 or 2, wherein the subject having immunosuppression therapy is a subject receiving a treatment with one or more immunosuppressants in order to prevent or lower the immune response of its body against a cell, tissue or organ allo- or xeno-transplant in a graft recipient, or auto-antigens in a subject suffering from an autoimmune disease or disorder.

4. An S1P receptor modulator or agonist according to claim 3, wherein the subject having immunosuppression therapy is a subject suffering from multiple sclerosis, psoriasis, asthma, lupus nephritis, rheumatoid arthritis or inflammatory bowel disease.

5. An S1P receptor modulator or agonist according to any preceding claim, wherein the S1P receptor modulator or agonist is selected from 2-amino-2-[2-(4-octylphenyl) ethyl]propane-1,3-diol (FTY720) in free form, in a pharmaceutically acceptable salt form, and FTY720-phosphate.

6. An S1P receptor modulator or agonist according to any one of claims 1 to 4, wherein the S1P receptor modulator or agonist is 2-amino-2-[2-(4-octylphenyl) ethyl]propane-1,3-diol (FTY720) in free form or in a pharmaceutically acceptable salt form.

7. An S1P receptor modulator or agonist according to claim 6, wherein the S1P receptor modulator or agonist is 2-amino-2-[2-(4-octylphenyl) ethyl]propane-1,3-diol in form of the hydrochloride.

8. A pharmaceutical combination comprising a) a first agent which is an S1P receptor modulator as defined in any one of claims 5 to 7, and b) a co-agent which has anti-HCV activities or an anti-viral agent.

9. A pharmaceutical combination according to claim 8, wherein the co-agent b) is selected from interferons, antiviral agents, thiazolidine derivatives, protease inhibitors, nucleoside and non-nucleoside inhibitors of HCV NS5B RNA-dependent RNA polymerase, nucleotide polymerase inhibitors, gliotoxin, cerulenin, helicase inhibitors, antisenses, inhibitors of IRES-dependent translation, and ribozymes.

10. A kit comprising a) a first agent which is an S1 P receptor modulator as defined in any one of claims 5 to 7, and b) at least one second drug substance, said second drug substance being as defined in claim 8 or 9.
